Europäisches Patentamt

European Patent Office

Office européen des brevets

(11) Publication number: **0 392 845**
**A2**

## (12) EUROPEAN PATENT APPLICATION

(21) Application number: 90303959.2

(22) Date of filing: **11.04.90**

(51) Int. Cl.⁵: **A61K 47/02, A61K 9/70, A61L 15/00**

(30) Priority: **14.04.89 US 336621**

(43) Date of publication of application:
**17.10.90 Bulletin 90/42**

(84) Designated Contracting States:
**CH DE ES FR GB IT LI NL SE**

(71) Applicant: **MINNESOTA MINING AND MANUFACTURING COMPANY**
**3M Center, P.O. Box 33427**
**St. Paul Minnesota 55133(US)**

(72) Inventor: **Dunshee, Wayne K., C/o Minnesota Mining**
**Manufacturing Company, 2501 Hudson Road**
**St. Paul, Minnesota 55133-3427(US)**
Inventor: **Bergsten, Ronald E., C/o Minnesota Mining**
**Manufacturing Company, 2501 Hudson Road**
**St. Paul, Minnesota 55133-3427(US)**

(74) Representative: **Baillie, Iain Cameron et al**
**c/o Ladas & Parry Isartorplatz 5**
**D-8000 München 2(DE)**

(54) **Solid gel external drug delivery system.**

(57) External drug delivery systems are described that deliver drugs that are oil soluble and water insoluble or sparingly water soluble, either topically or transdermally. These drugs are incorporated in a solid gel.

*FIG.1*

## SOLID GEL EXTERNAL DRUG DELIVERY SYSTEM

Field of Invention

The present invention relates to topical and transdermal drug delivery systems. More particularly, it relates to compositions and methods of using compositions to deliver oil soluble and water insoluble or sparingly water soluble drugs across the skin barrier using a flexible solid gel.

Background

Hydrogels have been used to deliver water soluble drugs in a time release manner in oral delivery systems and across the skin barrier.

U.S. Patent No. 4,318,746 to Claffey et al. discloses a "firm, cohesive and adhesive" gel made by combining a polymer that dissolves in hot water and forms a rigid gel on cooling with water and heating. A second polymer which is hot water insoluble is then added and the mixture is cooled to form a gel by change in physical state. Applicants have found by reproduction of the gel as taught that the resulting gel is thick and sticky, having a consistency similar to petrolatum. The incorporation of oils and other water-insoluble ingredients into the gel by emulsifying or dispersing them into the gel before it is cooled is described at column 7, lines 15 to 19. The gels must be prepared using carefully controlled heat to avoid dehydration, caking or premature gelation.

U.S. Patent No. 3,641,237 to Gould et al. discloses zero order delivery of water soluble pharmaceutically active organic compounds either alone or absorbed or encapsulated in hydrophilic polymers by providing a film diffusion barrier of an alkoxyethyl acrylate or methacrylate polymer. The diffusion barrier films can be employed in a laminate construction to provide a device for controlled zero order release of water soluble drugs for topical application. The water soluble pharmaceutical is protected from atmospheric oxygen by being entrapped in an inner matrix of a water insoluble and water swellable hydrophilic polymer, which is sealed in an all-encompassing, outer, drug diffusion rate-controlling thin film that is a polymer of an alkoxyethyl acrylate or methacrylate, optionally copolymerized with a hydrophilic polymer.

U.S. Patent No. 3,520,949 to Shepherd et al. discloses the preparation of a solid hydrophilic polymeric body by mixing in the solvent-free state a water soluble polymerizable hydroxyalkyl monoester of a mono-olefinic monocarboxylic acid with a comparatively minor amount of a polymerizable diester of a mono-olefinic monocarboxylic acid having at least two esterifiable hydroxyl groups. To this mixture is added a major amount of a linear polyamide resin having repeating carbonamido groups in the presence of an amount of a free radical vinyl polymerization catalyst sufficient to polymerize same. These polymers may be cast into any rigid form or may be provided as a powder that can be reconstituted in solution at will to provide solutions which give concentrated flavors or essences. In another embodiment of the invention disclosed at column 6, lines 45-52, the hydrophilic gel materials may be employed in the form of a covering or bandage carrying medication which can be slowly released from the hydrophilic gel material. Example 20 discloses the incorporation of a 2% aqueous solution of Merbromin (an antiseptic) in a hydrophilic film cast onto a dacron mesh cloth. The dried bandage is immersed in water and contacted with the open wound, gradually releasing the antiseptic.

U.S. Patent No. 3,576,760 to Gould et al. discloses entrapping drugs, pesticides, flavoring agents or fragrances in a water soluble polymeric system comprising hydroxyalkyl acrylates and methacrylates. This patent teaches that materials which are not water soluble may be entrapped, provided that they are soluble in other solvents in which the disclosed copolymers are soluble. These solvents are listed in the paragraph bridging columns 2 and 3.

U.S. Patent No. 3,998,215 to Anderson et al. discloses electrically-conductive gel pads used with biomedical electrodes. These gel pads are prepared from hydrogels such as polyvinyl alcohol or natural gums, and provide an electrically conductive, conformable interface between the skin and electrodes placed thereon. The gel pad is formed by dipping a porous webbed material into an aqueous solution of a hydrogel former such as polyvinyl alcohol or natural gums, with subsequent dipping into a gel forming agent, such as a borate salt. Claim 6 of this patent provides for the incorporation of a fungicide in the gel patch, and example 1 specifically discloses the use of a water soluble fungicide.

U.S. Patent No. 4,136,250 to Mueller et al. discloses a water insoluble hydrophilic gel incorporating a terminal polyolefinic siloxane macromer. The disclosed uses of this material include the use as a carrier for biologically active substances. An active agent is incorporated in the hydrogel by swelling the hydrogel in an appropriate solvent containing the agent to be delivered. The solvent is then evaporated, leaving the agent within the hydrogel particles. Upon contact with an aqueous environment, the agent is released in a controlled manner.

U.S. Patent No. 4,439,584 to Gould et al. discloses gas and ion permeable membranes formed of polyurethane diacrylate compositions. The compositions may be mixed with or used to encapsulate drugs prior to the curing step. The drug is totally released when the polymer is placed in an aqueous or saline solution or in body fluids.

U.S. Patent No. 4,528,365 to Graham discloses a hydrogel which comprises polymerized moieties derivable from at least one polymerizable cyclic (thio)ether and at least one hydrophilic homo- or copolymer. The prepared hydrogels are disclosed to be usable in a controlled release composition comprising an active substance and the hydrogel. As taught at column 11, lines 38-47, the active substance must be soluble to an extent commensurate with the desired concentration in the controlled release composition in water or the organic solvent used to swell the hydrolizable hydrogel carrier. The use of this composition as a topical patch is disclosed at column 15, lines 19 and 20.

U.S. Patent No. 4,593,053 to Jevne et al. discloses an adhesive hydrogel composition comprising polyvinyl pyrrolidone, polyvinyl alcohol, a humectant, water, and optionally an ionic species or drug. The ionic species is provided in the hydrogel if the hydrogel is intended to be used to maintain an electrocardiogram electrode in place. When a drug is present, the gel is used as an iontophoresis device from which a drug is iontophoretically delivered.

U.S. Patent No. 4,638,043 to Szycher et al. discloses a drug releasing system in the form of a medical patch comprised of a drug dispensing polyurethane acrylic copolymer. As disclosed at column 4, lines 24 and 25, the drug must be water soluble.

U.S. Patent No. 4,659,700 to Jackson discloses a chitosan-glycerol-water gel which may be used as a carrier for a medicament that is customarily administered topically.

U.S. Patent No. 4,668,564 to Orchard discloses a hot or cold compress which is a layer of substituted urea/urethane hydrogel material bonded to a porous substrate. The additional incorporation of medication or other additives such as methyl salicylate is disclosed at column 5, lines 14-22.

U.S. Patent 4,725,439 to Campbell et al. discloses a medical device for the transdermal delivery of an active agent through sensitive intact skin. The active ingredient is contained within a "reservoir" which is a non-aqueous polymeric carrier selected from materials known in the art as is described at column 3, lines 40-53.

U.S. Patent No. 4,749,576 to Lee discloses a controlled release diffusion device comprising a swellable hydrogel matrix interpenetrated with an active agent having a concentration distribution increasing in a gradient normal to the surface of the device. The hydrogels that are used are water swellable but water insoluble polymers generally known in the art as described at column 4, line 63 through column 5, line 45, and including polyvinyl alcohol at column 5, line 42. The drugs to be incorporated must be soluble in the aqueous environment of use, as discussed at column 7, lines 34-43.

U.S. Patent No. 4,761,288 to Mezei discloses a pharmaceutical composition comprising a mixture of a) multilamellar lipid vesicles with a slightly water soluble biologically active compound as both a saturated aqueous solution and in solid form captured therein, b) a saturated solution of the biologically active compound, and c) the biologically active compound in solid form. This multiphase drug delivery system is disclosed as providing a sustained and prolonged action of the drug. This composition may be dispersed in a hydrocolloid gel, which influencing the structure and interrelationship of the lipid vesicles and affects the viscosity and adhesive properties of the final product. Hydrocolloids that may be used in the disclosed system are discussed at column 6, lines 25-30 and at column 9, lines 55 and 56.

U.K. Patent 1,108,837 discloses the topical administration of local anesthetic agents such as lidocaine by incorporating the local anesthetic agent in a water soluble, film-forming compound by solution or at least in extremely fine or colloidal dispersion. The film-forming compounds that may be used are listed on page 2 and include polyvinyl alcohol at line 15 and vegetable gums at line 31. The film may be laminated or covered with a water insoluble impermeable or resistant backing material such as plastic, cloth, metal foil or combinations thereof, and may be united to the water soluble film by lamination, heat, adhesives, pressure, or other suitable mechanical procedures or by wet casting a suitable liquid film-forming composition on such water insoluble backing so that adhesion is effected on drying. The local anesthetic is delivered by dissolution of the water soluble film on the skin.

PCT Patent No. WO 87/01973 to Bordoloi et al. discloses a system for incorporation of a heat sensitive material within a pressure sensitive adhesive for controlled release of the heat sensitive material. In this system, a fluid comprising a heat sensitive material and a prepolymer which, when subjected to the action of ultraviolet or electron-beam radiation is polymerized, is coated onto a surface. The prepolymer is then polymerized without thermal degradation of the heat sensitive material, so as to form a pressure sensitive adhesive product in which the material to be released is contained in a matrix of the formed pressure sensitive adhesive.

Japanese Patent 59-204,117 as described in the corresponding Chemical Abstracts, CA 102:119649h (1985) discloses topical medications for treatment of burns containing aloe extracts and hydrophilic water-containing gel bases such as polyvinyl alcohol polymers. The resulting mixture is spread on an unwoven rayon sheet to obtain a topical medication.

Until now there has not been a satisfactory method of transdermal or topical delivery of oil soluble and water insoluble or sparingly water soluble drugs. Creams, lotions, gels and ointments are of limited utility because these products allow the volatile active ingredients to evaporate to the air, thus limiting the length of time that the drug is available. These delivery systems also do not provide a controlled release of the medication to the treatment area. Drug delivery patches formulated from crosslinked polymers can contain undesirable free monomers and crosslinkers in the patch.

Summary of the Invention

An external drug delivery system is provided that is a solid gel easily prepared from a colloidal suspension containing an oil soluble and water insoluble or sparingly water soluble drug. A preferred drug delivery system delivers an analgesic drug such as methyl salicylate or menthol or a combination of the two for relief of muscular or arthritic pain. The solid gel is based on polyvinyl alcohol polymers or certain natural gums that have been gelled by inorganic gelling means.

As one particular aspect of this invention, it has been discovered that a stable emulsion system with polyvinyl alcohol polymers and containing methyl salicylate or the combination of methyl salicylate with menthol may be prepared for later gelling without the use of an emulsifier.

Brief Description of Drawings

Fig. 1 is a plan view of a solid gel patch composite of the present invention.
Fig. 2 is a cross-sectional view of the solid gel patch composite of Fig. 1 taken along lines 2-2.
Fig. 3 is an edge view of an alternative solid gel patch composite of this invention.
Fig. 4 is a perspective view of a solid gel patch composite of the present invention having adhesive domains.

Detailed Description of the Drawings

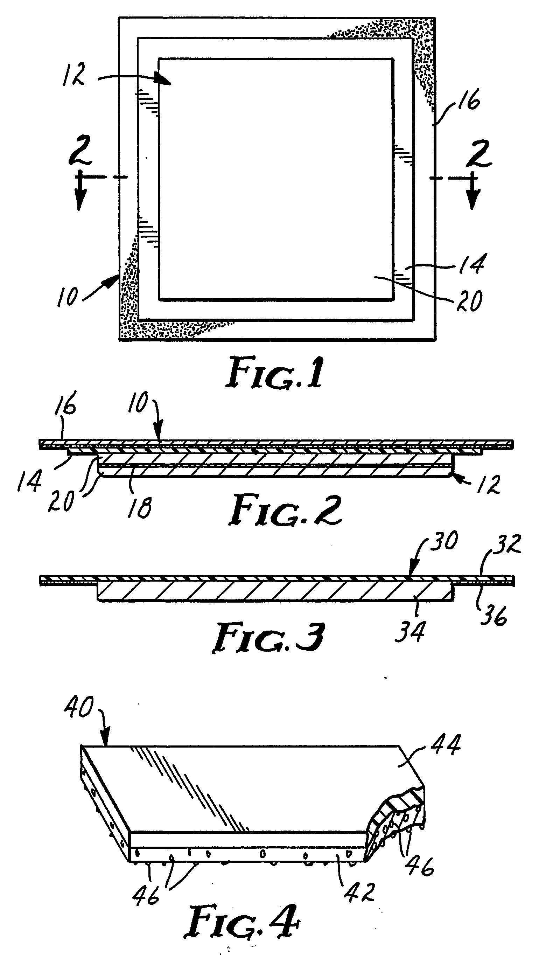

Figs. 1 and 2 show plan and cross-sectional views, respectively, of a solid gel patch composite **10** embodiment of the present invention, wherein like numerals refer to like parts. Solid gel patch composite **10** comprises solid gel patch **12**, vapor barrier **14** and adhesive coated sheet **16**. Solid gel patch **12** comprises gel **20**, made from materials as described herein, and support member **18**, which is made from cloth or other porous substrate or is made from a thermoplastic material or similar non-porous substrate. Vapor barrier **14** is made from a thermoplastic material and covers one side of solid gel patch **12** in order to reduce loss of water or active ingredient to the air. Preferably, vapor barrier **14** is larger than solid gel patch **12** and extends beyond it on all sides in order to prevent loss of water or active ingredient to the air around the edges of solid gel patch **12**. Adhesive coated sheet **16** is applied with adhesive coated side contacting vapor barrier **14**. Adhesive coated sheet **16** is larger than both vapor barrier **14** and solid gel patch **12** and extends beyond them on all sides so that the edge portions of adhesive coated sheet **16** may contact the skin upon application to the patient.

Fig. 3 is an edge view of an alternative solid gel patch composite **30** of the present invention. Support member **32** is made from a thermoplastic material and simultaneously acts as a vapor barrier. Gel **34** is preferably cast in the center area of support member **32** only, with adhesive **36** being applied to the periphery of support member **32** on the same side as gel **34**. Solid gel patch composite **30** is applied to the skin of a patient so that gel **34** contacts the skin and adhesive **36** will hold composite **30** in place.

Fig. 4 is a perspective view of solid gel patch composite **40** of the present invention. Gel **42** is cast on support member **44**, which is preferably made from a thermoplastic material so that it also acts as a vapor barrier. Disposed within gel **42** are adhesive domains **46**, which are micelles of varying sizes containing adhesive as described herein. Sufficient adhesive is available on the skin contacting surface of gel **42** so that solid gel patch composite **40** will adhere to the skin without the use of an adhesive coated sheet.

Detailed Description

It has been surprisingly discovered that bio-active agents may be incorporated in desirable and effective concentrations in a solid hydrogel system that has been gelled from a colloidal suspension of hydrogel polymers using inorganic gelling means. Although such solid hydrogel patches have been utilized as bio-medical electrode conductive gel pads as disclosed in U.S. Patent No. 3,998,215, only very small amounts of water soluble fungicides have been incorporated therein as a preservative for the pad. Attempts to incorporate more than about 2% by weight of water soluble active ingredients based on total composition weight for topical delivery to the body result in interference with the gelling of the colloidal suspension. Less than about 2% of active ingredient is at best at the bottom range of FDA approved concentrations of most topically applied drugs, and is much less than the optimal drug concentration capability for a desirable external drug delivery system.

An oil soluble and water insoluble or sparingly water soluble active ingredient can now be incorporated in amounts up to about 60% into a solid gel made from a colloidal suspension gelled by inorganic gelling means. The resulting solid gel has sufficient internal strength that it is capable of being removed from the skin as a single sheet without leaving hydrogel residue, even though between about 5 and 60% of the patch is actually oil and therefore does not contribute to the hydrogel matrix.

Preferably, the solid gel of the present invention comprises between about 10 and 50% active ingredient, and more preferably between about 15 and 30% active ingredient. It is surprisingly possible to achieve the desirable properties of the solid gel with these high active ingredient concentrations even when preparing the gels from colloidal suspensions having relatively low concentrations of hydrogel polymers. Thus, solid gels may be formed from suspensions containing less than 10% or even less than 5% polyvinyl alcohol by weight. Preferably, the suspension contains about 8% polyvinyl alcohol.

The external drug delivery system of the present invention provides excellent delivery of an oil soluble and water insoluble or sparingly water soluble drug through the skin. This delivery may be provided for either short term applications or for applications lasting in excess of four or even eight hours. By selection of active ingredients present in appropriate quantities, the external drug delivery system of the present invention may provide either topical or transdermal delivery of the active ingredient.

The solid gel of the present invention is prepared from a colloidal suspension using hydrogel polymers that are gelled by inorganic gelling means. Examples of these hydrogel polymers include polyvinyl alcohol polymers and some natural gums such as guar gum, alginate gum, pectic acid gum or blends of natural gums. The gelling means is selected according to its ability to gel the specific hydrogel polymer to be used in the solid gel of the present invention, and is preferably an inorganic compound having minimal toxicity and irritation to humans. Thus, polyvinyl alcohol, locust bean gum, guar gum and other hydrogels having cis hydroxyl groups available for reaction are gelled safely and effectively to a solid state by borate compounds such as borate salts, boric acid or borax. Preferably, the gelling means is potassium tetraborate. Similarly, alginate gum and pectic acid gum are gelled safely and effectively by calcium compounds such as calcium citrate or calcium chloride.

While not wishing to be bound by theory, it is believed that gelling of the hydrogel polymers results in binding up or encapsulating the active ingredient within the matrix of the resulting solid gel. As the gel hydrates the skin, the patch loses some of its volume and essentially squeezes out the active ingredient from the gel matrix, thereby delivering the active ingredient to the skin surface. Because the skin has been hydrated by the gel, the active ingredient may diffuse through the upper layers of the skin and is made available for action on the body. Thus, the active ingredient is bound within the system so that it is not lost to the air and is simultaneously provided at a relatively controlled release rate to the user.

The preferred hydrogels used in the colloidal suspension to form the solid gel are polyvinyl alcohol polymers. These polyvinyl alcohol polymers are preferably of medium to low molecular weight. While not wishing to be bound by theory, these polymers appear to provide relatively short chains from which the solid gel matrix is formed. In this manner, the ultimate gel tends to "tie up" water better because of the multiple links and the relatively smaller cavities formed by the gel matrix.

The polyvinyl alcohol starting material preferably has between about 87 and 100 mole percent hydrolysis of the acetate groups. Although residual acetate-group content is not critical in the practice of this invention, C. A. Finch reports in "Polyvinyl Alcohol" (John Wiley & Sons, 1973) that gelling becomes easier as the residual content increases.

Preferably, the polyvinyl alcohol colloidal suspension at 4% solids has a viscosity of at least about 5

5

centipoise. This viscosity provides sufficient colloid to gel and form the intended matrix. A viscosity of higher than about 50 centipoise is difficult to process.

When the gelling means requires the presence of cis hydroxyl groups in the hydrogel, a highly syndiotactic polyvinyl alcohol polymer is inappropriate for use as the sole hydrogel present in a colloidal suspension used to formulate the solid gel. Atactic polyvinyl alcohol polymers, polyvinyl alcohol polymers of various degrees of tacticity in the chain, blends of polyvinyl alcohol polymers of different tacticities and copolymers that are predominantly alcoholic in nature are contemplated to be appropriate when such gelling means is used.

The active agents to be used in the external drug delivery system of the present invention may consist of any oil soluble and sparingly water soluble or water insoluble drugs. Stating the same concept in another way, the active ingredients are either oil soluble and water insoluble or are oil soluble and sparingly water soluble. Such active agents may include, for example, the antifungal haloprogin, the antiseptics hexylresorcinol and parachlorometaxylenol, the skin care drug vitamin A palmitate or menadione, the external analgesics eucalyptus oil, methyl salicylate, menthol, camphor or caspicum or the topical anesthetics lidocaine and benzocaine. In addition to the above recited drug component, the external drug delivery system may incorporate small amounts of water soluble active compounds, provided that the emulsion and the ultimate gel are not adversely affected by their presence.

Emulsifiers to be used in the gel patch of this invention should be anionic or non-ionic in character to avoid adverse interaction with the gelling means when it is an organic compound, and should have an HLB value of less than 40. Examples of emulsifiers include oleth 20, polyoxyethylene 21 stearyl ether, polyoxyethylene 2 stearyl ether (commercially available as BRIG 721 and BRIG 72, respectively, from ICI Americas, Inc., Wilmington, DE), and poloxamer 407 or mixtures of emulsifiers.

Additional components may be incorporated in the system of this invention such as dispersants, preservatives, stabilizers, colorants, perfumes and penetration enhancers. Dispersants facilitate the soublization of the surfactant or some active ingredients throughout the gel patch. An example of a dispersant is S.D.A. 40 denatured alcohol. An example of a preservative is Germaben II™, commercially available from Sutton Laboratory, Inc., Chatham, NJ. An example of a stabilizer is a natural gum that does not gel under the conditions of the present invention, such as xanthan gum. An example of a penetration enhancer is propylene glycol.

The solid gel of the present invention is made by preparing a colloidal suspension water phase and a separate oil phase, with active ingredients and additional components added to the appropriate phase. The two phases are then combined according to techniques well known in the emulsion chemisty art. If insufficient oil phase volume to support an emulsion is provided by the oil soluble components, the addition of a neutral oil may be necessary to sucessfully prepare the emulsion. Examples of neutral oils include mineral oil (such as the Finsolv™ products commercially available from FineTex Inc., Elmwood Park, NJ), olive oil or other vegetable oils.

If the colloidal suspension containing all of the ingredients of the ultimate solid gel patch is to be gelled within about five minutes, no emulsifier is required to stabilize the oil and water phases of the intermediate solution. Rapid mixing of the oil and water phases using techniques of emulsion preparation followed by addition of the gelling means will form a stable solid gel patch without addition of any emulsifier. When gelling of the emulsion within about five minutes is not feasible, the use of an emulsifier in the colloidal suspension containing an oil phase is generally required.

It has been surprisingly discovered that in the particular case of the use of methyl salicylate in polyvinyl alcohol, a stable emulsion system may be prepared by using a stabilizer such as xanthum gum, without the use of an additional emulsifier. Instead, methyl salicylate with a small amount of alcohol is added as one would add an oil phase in the preparative discussion above. Without being bound by theory, it appears that methyl salicylate is stabilized by the polyvinyl alcohol and acts as its own emulsifier in this particular system.

Gelling means is added to the resulting emulsion in an amount effective to cause the formation of a solid gel. The amount of gelling means that is effective may be readily determined through routine experimentation by the practitioner. A solid gel is defined as a material which, when provided as a 3 cm by 3 cm by 1 mm patch and placed on a stainless steel ruler at a 60 degree angle in a 100% relative humidity environment, will show a change in the contact angle (angle between the ruler and the down-slope edge of the patch) of no more than about 90 degrees and little or no movement of the gel patch down the ruler after a 24 hour period.

The emulsion may be coated on a support member before addition of the gelling agent. This support member may be an non-porous substrate such as a thermoplastic material (e.g., polyester film), or a porous substrate such as a woven or nonwoven fabric. Depending on the coating technique used, the support member may be located in the middle of the solid gel or on one side. When the support member is a thermoplastic material, it will also function as a vapor barrier to curb the loss of volatile active ingredients to the air. Examples of coating techniques that may be used are dip coating, curtain coating, extrusion coating, roll coating, hopper knife coating or spray coating. Selection of the desired coating weight may be assisted by adjusting relative proportions of ingredients in the composition, particularly the amount of xanthan gum stabilizer used.

The emulsion may also be gelled in a solid block and cut into patch sizes suitable for use as a drug delivery system.

More preferably, the emulsion is gelled as an integral component with a support member, which is a web of a porous substrate such as a woven fabric. In a preferred process method, the porous substrate is impregnated with a solution containing gelling means which is an inorganic compound of minimal toxicity, and allowed to dry. The porous substrate is then drawn through or coated with the emulsion described above to form the solid gel.

The solid gel of the present invention is provided in any size and thickness as may be appropriate for the particular intended use. For example, when the gel is intended to deliver analgesic drugs for the relief of muscular or arthritic pain, the gel patch should be of sufficient size to cover the intended area of treatment. When the gel is intended to deliver a drug such as an antibiotic transdermally, the area of the gel patch should be small so that it is unobtrusive to the user. The use of a small patch may require a higher concentration of active ingredient in the patch in order to provide sufficient quantities of active ingredient.

An additional alternative method of formation of the solid gel is by applying an emulsion cream to the skin, especially a hairy area, and spraying, painting or otherwise applying a liquid inorganic gelling means to the emulsion. The solid gel is thereby formed in place, which can be a particular advantage in application in heavy hair or fur areas that would otherwise impede contact of the solid gel with the skin. Topical treatment can thus easily be provided to areas where shaving to remove hair or fur is undesirable. This method is particularly advantageous for applications in the animal care field. Preferably, the emulsion cream comprises polyvinyl alcohol polymers. The liquid inorganic gelling means is preferably an aqueous solution containing a borate salt.

Because the emulsion is provided to the consumer before it is gelled, the product has a very good shelf life and by definition cannot experience syneresis. Because the emulsions of the present invention are remarkably stable, they will survive multiple freeze/thaw cycles before gelling.

The intended ultimate use of the solid gel is dictated primarily by the selection of active ingredient incorporated in the delivery system. When an analgesic or topical anesthetic is incorporated, the gel patch may be used to relieve local pain. When a skin care drug or similar agent is used, the patch may be utilized as a facial mask. When a pharmaceutical formulation for internal action is incorporated, the patch acts as a transdermal drug delivery system.

A concentration gradient of active ingredient may be provided in the solid gel patch, so that the active is delivered in a relatively constant rate. Such a concentration gradient would be produced by casting and gelling a first layer having a high concentration of active ingredient, with subsequent casting of layers having successively lower concentrations of actives. In use, the layer with the lowest concentration of active is applied directly to the skin, with the higher concentration layers being remote from the skin. A nearly zero order delivery rate of the active ingredient is provided through the migration of the active ingredient through the gel patch to the skin.

An additional embodiment of the present invention may incorporate emulsions containing pressure-sensitive adhesives so that the solid gel will be self-adhering to the skin. Such adhesive emulsions are disclosed, for example, in U.S. RE 24,906; U.S. 4,629,663; U.S. 4,645,711 and U.S. 4,699,842. Preferably, the adhesive emulsion is acrylate based. As the colloidal suspension gels, it pulls the water from the adhesive emulsion and forms solid adhesive domains on the surface of the solid gel. The gel can therefore be attached to the body without the use of additional adhesive tapes.

When an adhesive emulsion is used, protective means such as a polypropylene sheet may be provided on the side of the gel patch that does not contact the skin so that the adhesive gel patch does not catch on clothing, etc. In addition to protecting the adhesive, such protective means may provide a vapor barrier to curb the loss of volatile active ingredients to the air.

Care should be exercised in the selection of components to the ultimate composition to assure that there are no adverse interactions between the components. For example, methyl salicylate has been observed to dissolve the acrylate polymers in many adhesives. Methyl salicylate should therefore not be used with acrylate adhesive emulsions or with non-woven support materials prepared using acrylate adhesives.

The solid gel patch of the present invention is applied to the user by merely placing the patch on the selected area for treatment and taping it in place. It is contemplated that the adhesive tape to be used would be a single piece of medical grade tape having a slightly larger area than the gel patch so that the patch is adhered to the skin around the entire periphery of the solid gel and is also protected from exposure to the air. Additionally, a film made from thermoplastic material may be placed on the gel patch before overtaping to act as an additional vapor barrier. Alternatively, the thermoplastic film could itself be coated at its periphery or completely coated on one side by adhesive so that no separate adhesive tape would be required. Preferably, the gel patch, vapor barrier film and adhesive tape would be provided in a one piece package system so that the user could apply the gel patch as a one step procedure.

An alternative method of providing a vapor barrier is to coat the solid gel patch on the side that does not contact the skin with a "paint-on" coating such as a cyano acrylate adhesive before application of the patch to the skin.

An alternative method of preparing a gel patch of the present invention comprises providing to the user a tube of the emulsion described above and a separate sheet of fabric or other porous material that has been impregnated with gelling means. To use, the user merely applies the emulsion to the body and presses on the gelling means-impregnated sheet. The emulsion then gels in place on the body. Alternatively, the user may apply the emulsion to the gelling means-impregnated sheet, with subsequent application of the sheet to the treatment site. Again, the emulsion gels in place on the body, a process that takes anywhere between about 2 and 5 minutes.

Yet another method of preparing a gel patch of the present invention, as discussed above, is providing to the user a separate emulsion cream and a liquid inorganic gelling means adapted to gel this emulsion as a two-part kit. The user applies the cream to the desired location and sprays, paints on or otherwise applies the gelling means to the cream. The emulsion cream then gels in place on the body. A thermoplastic film, such as a polyester or polyethylene film, may optionally be placed on top of the thus formed patch so that it does not dry out due to evaporation of water. The patch may additionally be overtaped with a conventional medical adhesive tape or similarly bandaged to further protect the patch.

It is envisioned that under certain applications, the penetration of the active ingredient through the skin may be enhanced by applying heat to the outside surface of the patch. This may be accomplished by the use of a hot water bottle, a commercially available hot pack or a disposable self-heating plaster such as the Marukyu-ban™ plaster, available in Japan. The Marukyu-ban™ plaster provides heat to a site by a controlled thermal reaction when the plaster is exposed to air. The additional use of heat, of course, is dictated by the active ingredient to be used. For example, the use of heat with methyl salicylate and menthol is never advised because of the deleterious effects that this combination has on human tissue.

As an additional alternative method of providing the solid gel patch of this invention to the user, the patch may be packaged in a dehydrated state. It has been surprisingly discovered that gel patches made according to the present invention can retain useful concentrations of the active ingredient even after being dehydrated. The shelf life of the gel patch in this dehydrated state is much longer than the shelf life of the corresponding hydrated product. A dehydrated patch is provided by preparing the solid gel as described above and drying at ambient temperature or baking in an oven until dry at a temperature that does not adversely affect the active ingredient. In the case of gels containing methyl salicylate and menthol, this temperature may be about 22˚. The user then merely rehydrates the patch by dipping it in water and applying to the intended site as described above.

When provided in its hydrated state, the gel patch is preferably packaged in a highly water impermeable material, such as a foil package. A particularly preferred foil packaging system would utilize a polyethylene/foil/polyethylene laminate or a polyethylene/foil/polyethylene/paper laminate.

The invention is further illustrated by the following nonlimiting examples. All parts provided are by weight.

## PREPARATION OF EMULSION

### Example 1

To 14.73 parts of methyl salicylate and 4.91 parts of menthol was added 2.94 parts of oleth 20 and 4.91 parts of S.D.A. 40 denatured alcohol. This oil phase mixture was warmed slightly to provide complete mixing of the surfactant with the other components. In a separate container, 56.24 parts of D.I. water was mixed with 7.85 parts of Elvanol™ 51-05 polyvinyl alcohol, 5.90 parts of propylene glycol, 0.49 parts of

Keltrol T™ xanthan gum and 0.25 parts of Germaben II™ preservative. This water phase solution was heated to about 93°C and was agitated with a high shear mixer and cooled to about 70°C. The oil phase solution was heated quickly to about 70°C and was added steadily and quickly into the water phase with continued mixing for 15 minutes. The resulting emulsion was slowly allowed to cool. Water was then added with mixing in quantum· sufficient to return the total weight up to 98.13 parts due to water lost by evaporation.

Examples 2-3

Emulsions were prepared as in Example 1, except that the components were provided in amounts as follows:

|  | Example (parts) | |
|---|---|---|
| Ingredient Name | 2 | 3 |
| Oil Phase | | |
| S.D.A. 40 Denatured Alcohol | 4.91 | 4.91 |
| Oleth 20 | 3.93 | 2.96 |
| Methyl Salicylate | 14.73 | -- |
| Menthol | -- | 9.85 |
| Water Phase | | |
| D.I. Water | 60.18 | 66.21 |
| Elvanol™ 51-05 polyvinyl alcohol | 7.85 | 7.85 |
| propylene glycol | 5.89 | 5.90 |
| Keltrol T™ xanthan gum | 0.49 | 0.49 |
| Germaben II™ preservative | 0.25 | 0.25 |

## PREPARATION OF GEL PATCH

Example 4

The emulsion of Example 1 was coated on a polyester film by hopper knife coating to a thickness of approximately 25 mils. An aqueous solution containing potassium tetraborate was sprayed onto the cast emulsion to deliver about 1.5 parts of potassium tetraborate for gelling.

Example 5

The emulsion of Example 2 was cast on a film and sprayed with a solution to deliver about 1.5 parts of potassium tetraborate as in Example 4 above.

Example 6

The emulsion of Example 3 was cast on to a film and sprayed with a solution to deliver about 1.5 parts of potassium tetraborate as in Example 4 above.

## PREPARATION OF SOLID GEL PATCH WITHOUT ADDITON OF EMULSIFIER

## Example 7

Fifteen parts of methyl salicylate and 5 parts of S.D.A. 40 denatured alcohol were mixed together. In a separate container, 65.25 parts of DI water was mixed with 8 parts of Elvanol 51-05 polyvinyl alcohol, 6 parts of propylene glycol, 0.5 parts of Keltrol T™ xantham gum and 0.25 parts of Germaben II™ preservative. This water phase solution was heated to about 93°C and was agitated with a high shear mixer and cooled to about 70°C. The oil phase solution was heated quickly to about 70°C and was added steadily and quickly into the water phase with continued mixing for 15 minutes. The resulting emulsion was slowly allowed to cool. Water was then added with mixing in quantum sufficient to return the total weight up to 100 parts due to water lost by evaporation.

The resulting solution was then coated on a polyester film by hopper knife coating to a thickness of approximately 25 mils, and was sprayed with an aqueous solution to deliver about 1.5 parts of potassium tetraborate.

## PREPARATION OF EMULSION CONTAINING ADHESIVE

## Example 8

To 40 parts of an acrylate adhesive emulsion wherein the acrylate is a copolymer of butyl acrylate, methyl methacrylate, vinyl acetate and acrylic acid in the approximate relative ratio of 89:6:3:2 (commercially available as 2397 Acrylic emulsion from Monsanto, Inc., St. Louis, MO) that had been diluted to 25% solids is added 2.5 parts 5% solids by weight 51-05 Elvanol™ polyvinyl alcohol solution. This solution is heated to about 65°C. Under high shear mixing is added 7.5 parts of methyl salicylate.

The resulting solution was coated onto a dry cheese cloth that had been soaked in a 10% potassium borate solution by the hopper knife coating method in a thickness of about 25 mils. The solution congealed to form a solid gel that possessed adhesive properties.

| PREPARATION OF HIGH ACTIVE INGREDIENT CONCENTRATION EMULSIONS | | | | | | | | | |
|---|---|---|---|---|---|---|---|---|---|
| Emulsions were prepared as in Example 1, with the following active ingredient contents. | | | | | | | | | |
| | Examples (parts) | | | | | | | | |
| | 9 | 10 | 11 | 12 | 13 | 14 | 15 | 16 | 17 |
| Water Phase | | | | | | | | | |
| D.I. Water | 37.25 | 42.25 | 37.25 | 32.25 | 30.25 | 25.25 | 30.25 | 51.02 | 44.16 |
| Elvanol 51-05™ PVA | 8 | 8 | 8 | 8 | 5 | 5 | 5 | 4.76 | 4.12 |
| Keltrol T™ Xanthan Gum | 0.5 | 0.5 | 0.5 | 0.5 | 0.5 | 0.5 | 0.5 | 0.5 | .388 |
| Germaben II™ | 0.25 | 0.25 | 0.25 | 0.25 | 0.25 | 0.25 | 0.25 | 0.149 | 0.12875 |
| Propylene glycol | -- | -- | -- | -- | -- | -- | -- | 3.57 | 3.09 |
| Oil Phase | | | | | | | | | |
| Menthol | -- | -- | -- | -- | -- | -- | 10 | -- | -- |
| Methyl Salicylate | 50 | 45 | 50 | 55 | 60 | 65 | 50 | 36 | 34.17 |
| oleth 20 | 4 | 4 | -- | -- | -- | -- | -- | 4 | 3.11 |
| BRIG 721™ | -- | -- | 2 | 2 | 2 | 2 | 2 | -- | -- |
| BRIG 72™ | -- | -- | 2 | 2 | 2 | 2 | 2 | -- | -- |
| Examples 9 and 14 separated into two phases upon cooling. The same relative ratios of components as present in Example 9 were successfully utilized in Example 11 with a different emulsifier. Example 14 indicates that there may be an upper li it to the amount of oil that can be used in the present system. Examples 10-13 and 15 formed very satisfactory, thick emulsions. | | | | | | | | | |

Examples 10-13 and 15-17 were spread onto a dry 10 x 15 cm cheese cloth that had been immersed in a potassium tetraborate solution to provide about 0.52 grams of gelling agent per sample. These examples provided satisfactory solid gels.

## APPLICATION TO SKIN

5 cm. by 5 cm. samples were cut from the samples prepared in Examples 1-8. These samples were applied to volunteer human subjects' forearms, gel side down. 7 cm. by 7 cm. fabric backed tape was applied over the solid gel patch to secure the patch on the arm. Each of the patches provided a soothing, tingling and slightly warming feel to the subject for a period in excess of 4 hours each.

A 5 cm. by 5 cm. sample of the solid gel patch of Example 8 was placed on a volunteer human subject's forearm. Because this sample contained adhesive, no additional adhesive tape was required. This sample provided a soothing, tingling and somewhat warming sensation to the user.

## SYNERESIS TEST

In order to provide effective delivery of an active agent, a limited amount of syneresis (exudation of the liquid component of a gel) of the solid gel patch is desirable. However, an excessive amount of syneresis results in an unattractive and ineffective product. Thus, while a gel of the present invention will always undergo some syneresis, it is preferred that the solid gel exhibit no more than about 20% syneresis as defined below, and most preferably the solid gel will exhibit no more than about 10% syneresis as defined below. Using well established advanced aging techniques, the long term stability and effectiveness of the product may be evaluated. A typical accelerated aging model consists of storing the product in an oven set at about 38°C. In this model, a 3 month controlled storage time is deemed to be equivalent to about 2 years of normal shelf life.

Syneresis may be evaluated by initially weighing the solid gel patch together with the water impermeable packaging material after removing same from the accelerated aging oven. The patch is then removed from the package, and both the patch and the package are quickly blotted with a dry towel or swab to absorb any moisture thus available. The package and patch are both weighed again, and the dried package is weighed separately. The percent syneresis is defined as the percentage of weight loss of the tared patch.

## PREPARATION OF FORM-IN-PLACE CREAM

An emulsion was prepared as in Example 1, except that the components were provided in amounts as follows:

EP 0 392 845 A2

|  | Example 17 |
|---|---|
| Ingredient Name | % by weight |
| Oil Phase | |
| Methyl Salicylate | 15.00 |
| Menthol | 4.00 |
| BrijTM 721 emulsifier | 2.25 |
| BrijTM 72 emulsifier | 2.25 |
| Water Phase | |
| Water | 66.75 |
| ElvanolTM SI-05 polyvinyl alcohol | 8.00 |
| Propylene glycol | 1.00 |
| Keltrol TTM xanthan gum | 0.50 |
| Germaben IITM preservative | 0.25 |

This cream may be used with the gelling means-impregnated absorbent substrate (described in Examples 9-13 and 15-17) sheet or as a pre-formed gel provided on a substrate (described in Examples 4-7), as well as in the form-in-place application.

## APPLICATION OF FORM-IN-PLACE GEL TO SKIN

The cream of Example 17 is applied to the skin at about a 1.5 mm thickness over the entire area that treatment is desired. An aqueous solution of 17% potassium borate is sprayed onto the cream using an ordinary spray bottle in an amount effective to gel the cream. This amount is about 2 mls for every 10 mls of cream used.

The cream skins over immediately so that the gel is no longer sticky or tacky. The entire mass is completely gelled in place in about 10 minutes.

## Claims

1. A solid gel external drug delivery system comprising a natural gum or polyvinyl alcohol suspension and a drug that is oil soluble and water insoluble or sparingly water soluble that has been gelled using an inorganic gelling means.

2. The drug delivery system of claim 1 wherein said solid gel comprises polyvinyl alcohol polymers.

3. The drug delivery system of claim 1 wherein said solid gel comprises at least one natural gum.

4. The drug delivery system of claim 1 which comprises an analgesic.

5. The drug delivery system of claim 4 wherein said analgesic is methyl salicylate, and additionally comprising menthol.

6. The drug delivery system of claim 1 wherein said gelling means is a borate compound.

7. An emulsion for use in preparing a solid gel external drug delivery system of claim 1 comprising a non-gelled natural gum or polyvinyl alcohol suspension, an emulsifier and a drug that is oil soluble and water insoluble or sparingly water soluble.

8. An emulsion for use in the preparation of a solid gel external drug delivery system of claim 1 comprising water, a non-gelled polyvinyl alcohol suspension, xanthan gum, menthol and methyl salicylate.

9. A solid gel external drug delivery system that is a two-part kit comprising

a) an emulsion comprising a natural gum or polyvinyl alcohol suspension and a drug that is oil soluble and water insoluble or sparingly water soluble, and

b) a liquid inorganic gelling means adapted to gel the emulsion of part a).

Claims for the following Contracting State: ES.

12

1. Method of preparing a solid gel external drug delivery system comprising combining a natural gum or polyvinyl alcohol suspension and a drug that is oil soluble and water insoluble or sparingly water soluble and gelling using an inorganic gelling means.

2. A method of claim 1 wherein said solid gel comprises polyvinyl alcohol polymers.

3. A method of claim 1 wherein said solid gel comprises at least one natural gum.

4. A method of any one of claims 1 to 3 which comprises an analgesic.

5. A method of claim 4 wherein said analgesic is methyl salicylate, and additionally comprising menthol.

6. A method of any one of claims 1 to 5 wherein said gelling means is a borate compound.

7. A method of preparing an emulsion for use in preparing a solid gel external drug delivery system produced according to claim 1 comprising combining non-gelled natural gum or polyvinyl alcohol suspension, an emulsifier and a drug that is oil soluble and water insoluble or sparingly water soluble.

8. A method for preparing an emulsion for use in the preparation of a solid gel external drug delivery system produced as in claim 1 comprising combining water, a non-gelled polyvinyl alcohol suspension, xanthan gum, menthol and methyl salicylate.

9. A two-part kit for preparing a solid gel external drug delivery system that is comprising

a) an emulsion comprising a natural gum or polyvinyl alcohol suspension and a drug that is oil soluble and water insoluble or sparingly water soluble, and

b) a liquid inorganic gelling means adapted to gel the emulsion of part a).

*FIG.1*

*FIG.2*

*FIG.3*

*FIG.4*